(19) Europäisches Patentamt
European Patent Office
Office européen des brevets

(11) Publication number: **0 002 605**
**B1**

(12) # EUROPEAN PATENT SPECIFICATION

(45) Date of publication of patent specification: **30.12.81**

(51) Int. Cl.³: **C 07 D 501/36,**
A 61 K 31/545

(21) Application number: **78300801.4**

(22) Date of filing: **13.12.78**

(54) **Cephalosporin compounds and formulations containing them for use in the treatment of bacterial infections and the preparation of the cephalosporin compounds.**

(30) Priority: **20.12.77 US 862318**

(43) Date of publication of application:
**27.06.79 Bulletin 79/13**

(45) Publication of the grant of the European patent:
**30.12.81 Bulletin 81/52**

(84) Designated Contracting States:
**DE GB IT NL SE**

(56) References cited:
EP - A - 0 000 005
FR - A - 2 280 381
FR - A - 2 348 218

(73) Proprietor: **ELI LILLY AND COMPANY**
**307, East McCarty Street**
**Indianapolis Indiana 46206 (US)**

(72) Inventor: **Katner, Allen Samuel**
**219 W. 81st Street**
**Indianapolis Indiana 46260 (US)**

(74) Representative: **McVey, Kenneth William Henry, et al,**
**Lilly House 13 Hanover Square**
**London W1R 0PA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 002 605 B1

Courier Press, Leamington Spa, England.

# Cephalosporin compounds and formulations containing them for use in the treatment of bacterial infections and the preparation of the cephalosporin compounds

The invention relates to novel pharmaceutical antibiotics of the cephalosporin class.

A considerable research effort has been directed to the development of new cephalosporin compounds for the treatment of infectious diseases in man. Most recently it has been disclosed that certain cephalosporins bearing a 2-(2-aminothiazol-4-yl)-2-methoxyiminoacetamido substituent at C—7 and substituents including acetoxymethyl, carbamoyloxymethyl, (1-methyl-tetrazol-5-ylthio)-methyl and (1,3,4-thiadiazol-2-ylthio)methyl at C—3, exhibit activity against both Gram positive and Gram negative microorganisms (Belgian Patents 852,860, 852,971, 850,662 and 853,545, West German Offen. 2,704,712, and Japanese Application No. 125,826/1976).

The invention relates to a new class of cephalosporin compounds having a 2-(2-aminothiazol-4-yl)-2-(hydroxy or $C_1$—$C_4$ alkoxy)iminoacetamido group at C—7 and a 4-($C_1$—$C_4$ alkyl)-5-oxo-6-hydroxy-4,5-dihydro-1,2,4-triazin-3-ylthiomethyl group at C—3. The compounds of this invention exhibit excellent broad spectrum Gram positive and Gram negative antibiotic activity. The present compounds exhibit superior antibiotic activity, especially against Gram positive microorganisms, when compared with other cephalosporin compounds, including the recently disclosed cephalosporins having a C—7 2-(2-aminothiazol-4-yl)-2-alkoxyiminoacetamido substituent.

The compounds of this invention have the formula

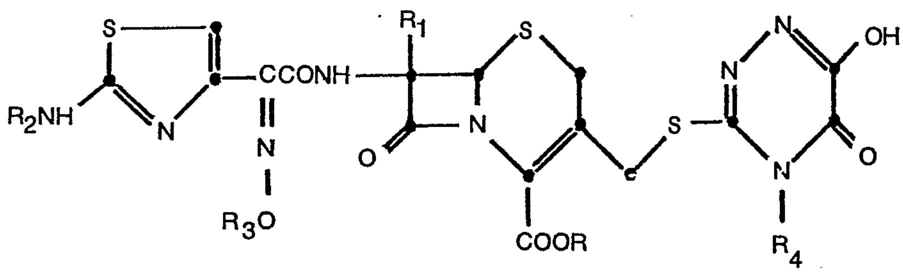

I

wherein R is hydrogen, an alkali metal cation, or a carboxylic acid protecting group; $R_1$ is hydrogen or methoxy; $R_2$ is hydrogen or an amino protecting group; $R_3$ is hydrogen or $C_1$—$C_4$ alkyl; and $R_4$ is $C_1$—$C_4$ alkyl.

The compounds of Formula I are prepared by a process comprising reacting a compound of the formula

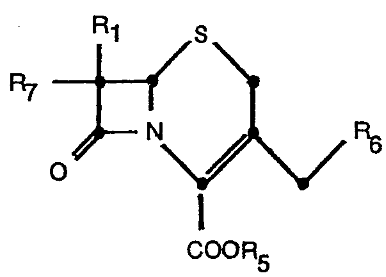

II

wherein $R_5$ is hydrogen, an alkali metal cation, or a carboxy-protecting group; $R_6$ is acetoxy, chloro, bromo, iodo or a group of the formula

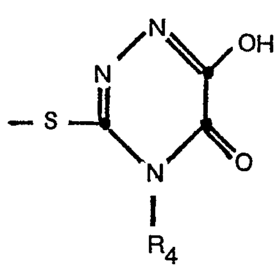

III

and $R_7$ is amino, amino substituted with a silyl amino-protecting group, amino hydrohalide or a group of the formula

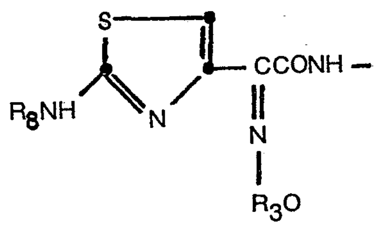

IV

wherein $R_8$ is hydrogen or an amino-protecting group; in either order with a thiol of the formula

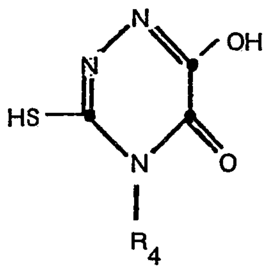

V

when $R_6$ is other than a group of formula III, and with an acylating agent of the formula

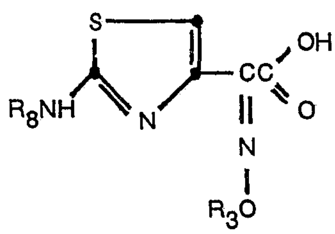

VI

which is in the form of the chloride, bromide or an activating ester when $R_7$ is other than a group of formula IV; optionally cleaving the amino-protecting and carboxy-protecting groups; and, when R does not represent a carboxy-protecting group, recovering the compound of formula I as the acid or the alkali metal salt.

In the above formula I $R_3$ is hydrogen or $C_1$—$C_4$ alkyl and $R_4$ is $C_1$—$C_4$ alkyl. By "$C_1$—$C_4$ alkyl" is meant methyl, ethyl, *n*-propyl, isopropyl, *n*-butyl, *sec*-butyl, isobutyl or *tert*-butyl. Preferably $R_3$ is methyl. Preferred groups represented by $R_4$ are methyl and ethyl. Most preferably $R_4$ is methyl.

Examples of the resulting C—3 substituent of the cephalosporin compounds of the present invention in which $R_4$ is as defined above include 4-methyl-5-oxo-6-hydroxy-4,5-dihydro-1,2,4-triazin-3-ylthiomethyl; 4-ethyl-5-oxo-6-hydroxy-4,5-dihydro-1,2,4-triazin-3-ylthiomethyl; 4-*n*-propyl-5-oxo-6-hydroxy-4,5-dihydro-1,2,4-triazin-3-ylthiomethyl; 4-isopropyl-5-oxo-6-hydroxy-4,5-dihydro-1,2,4-triazin-3-ylthiomethyl; 4-*n*-butyl-5-oxo-6-hydroxy-4,5-dihydro-1,2,4-triazin-3-ylthiomethyl; 4-*sec*-butyl-5-oxo-6-hydroxy-4,5-dihydro-1,2,4-triazin-3-ylthiomethyl; and 4-isobutyl-5-oxo-6-hydroxy-4,5-dihydro-1,2,4-triazin-3-ylthiomethyl.

In the above formula R is hydrogen, an alkali metal cation, or a carboxylic acid-protecting group. The term "a carboxylic acid-protecting group" refers to any group used to block or protect the cephalosporin C84 carboxylic acid functionality while reactions involving other functional sites are carried out. Such carboxylic acid-protecting groups are noted for their ease of cleavage, as for example by hydrolytic or hydrogenolytic methods to the corresponding carboxylic acid. Examples of suitable carboxylic acid-protecting groups are *tert*-butyl, 1-methylcyclohexyl, benzyl, 4-methoxybenzyl, 4-nitrobenzyl, acetoxymethyl, 1-acetoxyethyl, pivaloyloxymethyl, 1-pivaloyloxyethyl, carboethoxyoxymethyl, 1-carboethoxyoxyethyl, phthalidyl, 2-iodoethyl, 2-bromoethyl, benzhydryl, phenacyl, 4-halophenacyl, dimethylallyl, 2,2,2-trichloroethyl, methoxymethyl, tri($C_1$—$C_3$ alkyl)silyl and succinimidomethyl. Other known carboxylic acid-protecting groups are described by E. Haslam in "Protective Groups in Organic Chemistry," J. F. W. McOmie, Ed., Plenum Press, New York, 1973, Chapter 5. The nature of such groups is not critical; however, because of availability, ease of handling and other desirable properties, certain carboxylic acid-protecting groups are preferred. A preferred selection of carboxylic acid-protecting groups includes acetoxymethyl, 1-acetoxyethyl, pivaloyloxymethyl, 1-pivaloyloxyethyl, carboethoxyoxymethyl, 1-carboxyethoxyoxyethyl and phthalidyl. Another preferred group of carboxy-protecting entities comprises benzhydryl, 4-nitrobenzyl and *tert*-butyl.

Most preferred of the groups represented by R is hydrogen; the free acids represented by the

compounds in which R is hydrogen and the corresponding alkali metal salts, such as the sodium, potassium and lithium salts, are the most active of the compounds of the present invention. Compounds of the present invention where R is a carboxylic acid-protecting group are useful primarily as intermediates to the free acids and their alkali metal salts. In addition to serving as intermediates to the free acids, however, the present compounds wherein R is acetoxymethyl, 1-acetoxyethyl, pivaloyloxymethyl, 1-pivaloyloxyethyl, carboethoxyoxymethyl, 1-carboethoxyoxyethyl and phthalidyl show enhanced absorbability, resulting in higher blood levels, over the free acids of the present invention when employed in non-parenteral antibiotic administrations.

$R_2$ in the above formula representing the compounds of formula I is hydrogen or an amino-protecting group; hydrogen is preferred. The term "an amino-protecting group" refers to those groups which can be employed to block or protect the amino group while reactions involving other functional sites are carried out. Many amino-protecting groups and their preparation and properties are known to those skilled in the art. Examples of suitable amino-protecting groups are chloroacetyl, 4-nitrobenzyloxycarbonyl, 2,2,2-trichloroethoxycarbonyl, *tert*-butyloxycarbonyl, benzyloxycarbonyl, and trityl. Like conventional amino-protecting groups such as those described by J. W. Barton in "Protective Groups in Organic Chemistry," J. F. W. McOmie, Ed., Plenum Press, New York, 1973, Chapter 2, are suitable. Preferred amino-protecting groups are tert-butyloxycarbonyl, benzyloxycarbonyl and trityl. Trityl is most preferred.

The C—7 side chain substituent on the compounds of formula I is a 2-[2-(protected)aminothiazol-4-yl-2-(hydroxy or $C_1$—$C_4$ alkoxy)iminoacetamido group. In regard to the configuration of the hydroxyamino group or the alkoxyimino group, in relation to the adjacent carboxamido functionality, the compounds can exist as the *syn* (cis) isomer or *anti* isomer. The *syn* isomers are preferred in the present invention. The *syn* configuration is structurally denoted as indicated in the following partial structure:

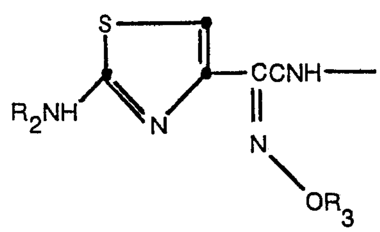

The following are exemplary of the C—7 side chain substituent of the present cephalosporin compounds:

2-(2-aminothiazol-4-yl)-2-methoxyiminoacetamido,
2-(2-aminothiazol-4-yl)-2-ethoxyiminoacetamido,
2-(2-tritylaminothiazol-4-yl)2-isopropoxyiminoacetamido,
2-(2-aminothiazol-4-yl)-2-*n*-propoxyiminoacetamido,
2-(2-*tert*-butyloxycarbonylaminothiazol-4-yl)-2-*sec*-butoxyiminoacetamido,
2-(2-benzyloxycarbonylaminothiazol-4-yl)-2-hydroxyiminoacetamido,
2-[2-(2-chloroacetamido)thiazol-4-yl]-2-methoxyiminoacetamido,
2-(2-aminothiazol-4-yl)-2-hydroxyiminoacetamido,
2-(2-tritylaminothiazol-4-yl)-2-ethoxyiminoacetamido,
2-[2-(2,2,2-trichloroethoxycarbonylamino)thiazol-4-yl]-2-isopropoxyiminoacetamido,
2-(2-aminothiazol-4-yl)-2-*n*-butoxyiminoacetamido,
2-(2-aminothiazol-4-yl)-2-isopropoxyiminoacetamido, and
2-(2-benzyloxycarbonylaminothiazol-4-yl)-2-methoxyiminoacetamido.

It should be noted that the compounds of the present invention can tautomerize both at the 2-aminothiazol-4-yl group on the C—7 side chain and at the 1,2,4-triazin-3-yl moiety at C—3. The possible tautomerization of each of these entities is illustrated by the partial structures below:

At C—7:

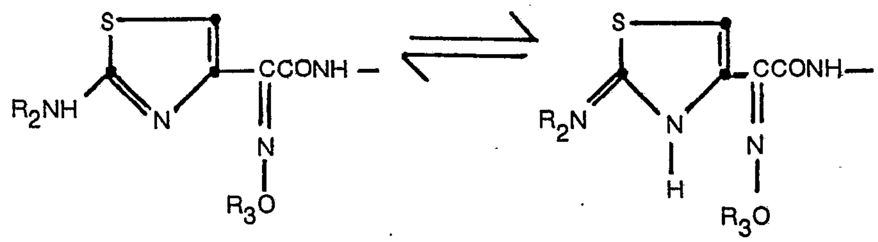

At C—3:

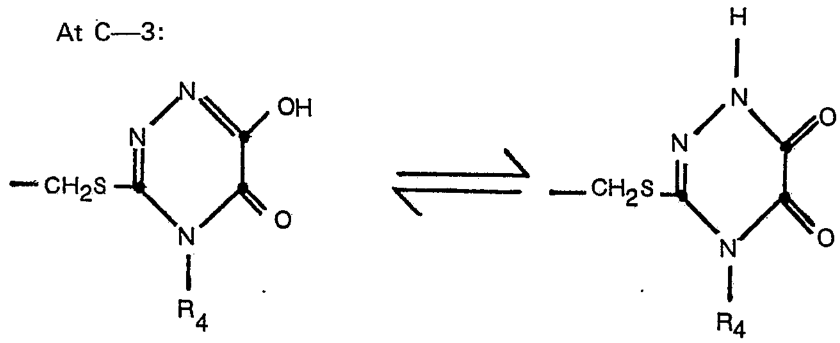

For the purposes of the present invention molecular structures, when drawn to one structure, shall be construed to represent also its tautomeric forms.

As has been briefly described, the compounds of formula I are prepared from a cephalosporin nucleus compound of formula II by the steps of acylation at the 7-position, and displacement at the 3-position, which steps may be carried out in either order. If a starting compound is available which bears either the triazinethiol of formula III or the aminothiazole group of formula IV, then only one of the steps of acylation and displacement will be necessary to prepare the desired compound of formula I. In general, the acylation and displacement steps proceed according to techniques which are established in cephalosporin chemistry.

The acylation step is carried out by reacting a nucleus compound of formula II wherein $R_7$ is amino, an aminohydrohalide or an amino group substituted with a silyl amino-protecting group, with an acylating agent of formula VI. The compound of formula II may be in the form of an acid, an alkali metal salt or an ester, as may be convenient in a given instance. Of course, the compound must be in the ester form if $R_6$ represents a halogen atom. If it is desired to use a compound of formula II in the ester form, then $R_5$ may represent any of the carboxy-protecting groups which have been discussed above, or it may also represent a silyl ester.

Silyl ester groups have been frequently described and used in the synthesis of cephalosporin compounds. They are easily provided by reacting the 4-acid group of a cephalosporin compound with a silylating agent which conveniently is a silazane such as N-trimethylsilylacetamide, trimethylchlorosilane, bis(trimethylsilyl)amine, trimethylsilylmethylamine, N,O-bis(trimethylsilyl)acetamide, N,O-bis(trimethylsilyl)trifluoroacetamide, N-trimethylsilyl-N-methyltrifluoroacetamide and N-trimethylsilylimidazole. The silyl esters form by contact of the acid with the silylating agent under mild reaction conditions.

The acylation step is carried out on compounds of formula II where the $R_7$ group is unsubstituted amino aminohydrohalide or amino substituted with a silyl group. The appropriate silyl groups are the same as those which have been described as appropriate silyl acid-protecting groups. Thus, if it is desired to carry out the acylation on a silyl-protected compound, it is highly convenient to react the desired cephem nucleus of formula II, in the acid form, where $R_7$ is unsubstituted amino, with a silylating agent, and thereby form the compound of formula II where both the 7-amino group and the 4-acid are protected with the same silyl group.

The 2-[2-(protected)aminothiazol-4-yl]-2-($C_1$—$C_4$ alkoxy or hydroxy)iminoacetic acids of formula VI used to acylate the aforedescribed 3-triazinylthiomethyl cephalosporin nucleus substrates are prepared in accordance with the procedures described in Belgian Patent No. 850,662. Generally the iminoacetic acids are derived from ethyl 2-acetyl-2-hydroxyiminoacetate by (1) optional alkylation of the hydroxyamino functionality; (2) bromination with bromine in methylene chloride at room temperature to provide ethyl 2-(α-bromoacetyl)-2-(hydroxyimino or $C_1$—$C_4$ alkoxyimino)acetate; (3) reaction with thiourea in aqueous ethanol to provide ethyl 2-(2-aminothiazol-4-yl)-2-(hydroxyimino or $C_1$—$C_4$ alkoxyimino)acetate; (4) blocking of the 2-amino group with an amino protecting group; and (5) base catalyzed deesterification.

The amino group of the compound of formula VI may be protected or not, as may be appropriate

in a given instance. If a protecting group is desired, it is chosen from the class of such groups which have been discussed above. Such groups have long been known in the cephalosporin art, as well as in general organic chemistry, and are easily added to the 2-amino group by trivial reaction steps.

The structure of the compound of formula VI is shown in the acid form, but it is actually used as an acylating agent, of course, by first converting it into the form of an acid chloride or bromide, or an activating ester group. Such activating esters include the esters formed with agents typified by dicyclohexylcarbodiimide, isobutyl chloroformate, 2-ethoxy-N-ethoxycarbonyl-1,2-dihydroquinoline, methyl chloroformate, ethyl chloroformate, hydroxypentachlorobenzene, N,N-diisopropylcarbodiimide, N-cyclohexyl-N'-(2-morpholinoethyl)carbodiimide and other related reagents which are now well known in the literature. Such activating esters are widely used in acylation reactions and have long been so used.

Acylations are carried out in inert organic solvents at temperatures in the range from about –20°C. to about 50°C. Temperatures from 0°C. to the ambient temperature are most convenient and are preferred. The solvent in which the acylation is carried out is entirely non-critical. Often, aqueous acetone is a particularly convenient solvent. In other instances, halogenated hydrocarbons such as dichloromethane, trichloroethane, chlorobenzene and the like are convenient. Ketones in general, including methyl ethyl ketone and methyl isobutyl ketone are useful, as are ethers including especially diethyl ether and tetrahydrofuran. Organic chemists will understand that the range of inert organic solvents usually used in reactions such as acylations can be used in these acylations as would be expected.

When the acylating agent is in the form of a halide, the reaction mixture should contain a hydrohalide acceptor or scavenger to improve the efficiency of the reaction. Simple bases such as tertiary amines and alkali metal hydroxides, carbonates and bicarbonates may be used as the hydrohalide acceptor in the usual fashion.

The compounds of formula II wherein $R_6$ is acetoxy are, of course, the well-known derivatives of cephalosporin C which are readily derived by fermentation. The compounds of formula II wherein $R_6$ is chloro, bromo, or iodo are prepared, for example by the halogenation process of U.S. Patent 4,042,585 from the corresponding 3-exomethylenecepham compounds.

The compounds of formula II wherein $R_1$ is methoxy can be prepared in accordance with the method of Koppel and Koehler, *J. Am. Chem. Soc. 95,* 2403 (1973). Such 7-methoxy compounds can also be prepared from 3-exomethylenecepham compounds, and a chloro or bromo $R_6$ group may be simultaneously put in place, by the methods of U.S. Patents 4,048,160 or 4,048,163.

As has been discussed, the compound of formula II wherein $R_7$ is a group other than the aminothiazole of formula IV, may be acylated either before or after the triazine group of formula III is attached at the 3-position of the cephalosporin molecule. In general, it is preferred to prepare the nucleus compound of formula II wherein $R_6$ is the triazine group of formula III, and to acylate as the final step of preparing the compounds of formula I. However, the order of the steps of displacement and acylation is not important, and the compounds are efficiently prepared in either way.

The displacement of an acetoxy, chloro, bromo or iodo group from the $R_6$ position of the compound of formula II is carried out according to literature procedures. For example, see Belgian Patent 831,787, showing the aqueous nucleophilic displacement of an acetoxy $R_6$ group and its replacement with the triazine of formula III. Such aqueous displacements are carried out at temperatures in the range of from 20°C. to 80°C. in aqueous reaction media which are preferably buffered at a pH in the range from 5 to 8.

Displacement of acetoxy $R_6$ groups may also be carried out, particularly on nucleus compounds of formula II which are acids wherein $R_5$ is hydrogen, under essentially anhydrous conditions by simple contact of the 3-acetoxymethyl compound of formula II with the triazinethiol of formula V. Such displacements are favorably carried out at temperatures from 50°C. to 140°C., in a great variety of solvents, including aromatic hydrocarbons, alkanoic acids, alkyl esters of alkanoic acids, nitroaromatics, nitroaliphatics, nitriles and ketones.

The triazine group of formula III may also readily be placed at the $R_6$ position of the compound of formula II by displacement of the halo $R_6$ group. Such displacements are carried out by reaction of the thiol of formula V with the nucleus compound where $R_6$ is halogen in an inert organic solvent at moderate temperatures such as from 0°C. to 50°C. The displacement requires only a hydrohalide scavenger, as discussed above, to proceed to high yields in moderate periods of time. Such a displacement is exemplified below.

The triazine of formula III may be placed on the nucelus compound of formula II which is either in a protected state, or bears the 4-acid group and the 7-amino group in an unprotected form. The displacement may be carried out in either way except, as previously described, the non-aqueous displacement of an acetoxy group should be carried out on a nucleus in the acidic form. Carboxy-protecting groups and amino-protecting groups have been discussed before.

The last step of the synthesis of the compounds of formula I is to remove $R_5$ carboxy-protecting groups and $R_8$ amino-protecting groups if such were used in the synthesis. Such groups, as has been stated before, are common in organic chemistry and are removed by the usual procedures For example, such carboxy-protecting groups are benzhydryl, *tert*-butyl and 4-methoxybenzyl are readily removed by

treatment with a strong acid such as formic or trifluoroacetic acid. Other carboxy-protecting groups, such as 2,2,2-trichloroethyl, are known to be readily removed by reduction as with zinc and acetic acid. Still other such groups, such as the 4-nitrobenzyl group, are most easily removed by hydrogenation such as in the presence of a noble metal catalyst.

Silyl carboxy-protecting groups, and silyl amino-protecting groups as well, are very conveniently removed by hydrolysis by simple contact of the protected compound with either water or an alcohol. Thus, both an amino-protecting group and a carboxy-protecting may be vary easily removed in a single step, when both are silyl groups.

Amino-protecting groups in general are removed by the methods known in the prior art. For example, amino-protecting groups such as trityl are readily removed by contact with a strong acid, particularly formic acid. Other commonly-used amino-protecting groups such as the chloroacetyl group are removed by contact of the protected compound with thiourea in the presence of a base. In general, amino-protecting groups are removed by the conventional hydrolytic means under mild conditions, and can often be removed in the same step which removes a carboxy-protecting group.

It is often advantageous to recover the product of formula I in the form of an alkali metal salt. Such salts are readily prepared in the usual way by simple contact of the acid with an alkali metal base, such as a hydroxide or carbonate of sodium, potassium or lithium, in a solvent such as aqueous acetone or an aqueous alcohol.

Exemplary of the biologically active 7-methoxy-cephalosporin compounds of the present invention are

7 - [2 - (2 - aminothiazol - 4 - yl) - 2 - methoxyiminoacetamido] - 7 - methoxy - 3 - (4 - methyl-5 - oxo - 6 - hydroxy - 4,5 - dihydro - 1,2,4 - triazin - 3 - ylthio)methyl - 3 - cephem - 4 - carboxylic acid,

7 - [2 - (2 - aminothiazol - 4 - yl) - 2 - hydroxyiminoacetamido] - 7 - methoxy - 3 - (4 - ethyl-5 - oxo - 6 - hydroxy - 4,5 - dihydro - 1,2,4 - triazin - 3 - ylthio)methyl - 3 - cephem - 4 - carboxylic acid,

7 - [2 - (2 - aminothiazol - 4 - yl) - 2 - ethoxyiminoacetamido] - 7 - methoxy - 3 - (4 - isopropyl-5 - oxo - 6 - hydroxy - 4,5 - dihydro - 1,2,4 - triazin - 3 - ylthio)methyl - 3 - cephem - 4 - carboxylic acid,

7 - [2 - (2 - aminothiazol - 4 - yl) - propoxyiminoacetamido] - 7 - methoxy - 3 - (4 - isopropyl-5 - oxo - 6 - hydroxy - 4,5 - dihydro - 1,2,4 - triazin - 3 - ylthio)methyl - 3 - cephem - 4 - carboxylic acid,

7 - [2 - (2 - aminothiazol - 4 - yl) - 2 - isobutoxyiminoacetamido] - 7 - methoxy - 3 - (4 - methyl-5 - oxo - 6 - hydroxy - 4,5 - dihydro - 1,2,4 - triazin - 3 - ylthio)methyl - 3 - cephem - 4 - carboxylic acid, and the corresponding lithium, potassium and sodium salts of each of the foregoing 7-methoxy cephalosporin compounds.

Exemplary of the biologically active compounds of the present invention wherein $R_1$ (in the above formula) is hydrogen are the following:

7 - [2 - (2 - aminothiazol - 4 - yl) - 2 - methoxyiminoacetamido] - 3 - (4 - ethyl - 5 - oxo - 6-hydroxy - 4,5 - dihydro - 1,2,4 - triazin - 3 - ylthio)methyl - 3 - cephem - 4 - carboxylic acid,

7 - [2 - (2 - aminothiazol - 4 - yl) - 2 - hydroxyiminoacetamido] - 3 - (4 - methyl - 5 - oxo - 6-hydroxy - 4,5 - dihydro - 1,2,4 - triazin - 3 - ylthio)methyl - 3 - cephem - 4 - carboxylic acid,

7 - [2 - (2 - aminothiazol - 4 - yl) - 2 - ethoxyiminoacetamido] - 3 - (4 - methyl - 5 - oxo - 6-hydroxy - 4,5 - dihydro - 1,2,4 - triazin - 3 - ylthio)methyl - 3 - cephem - 4 - carboxylic acid,

7 - [2 - (2 - aminothiazol - 4 - yl) - 2 - hydroxyiminoacetamido] - 3 - (4 - ethyl - 5 - oxo - 6-hydroxy - 4,5 - dihydro - 1,2,4 - triazin - 3 - ylthio)methyl - 3 - cephem - 4 - carboxylic acid,

7 - [2 - (2 - aminothiazol - 4 - yl) - 2 - methoxyiminoacetamido] - 3 - (4 - isopropyl - 5 - oxo-6 - hydroxy - 4,5 - dihydro - 1,2,4 - triazin - 3 - ylthio)methyl - 3 - cephem - 4 - carboxylic acid,

7 - [2 - (2 - aminothiazol - 4 - yl) - 2 - propoxyiminoacetamido] - 3 - (4 - *n* - butyl - 5 - oxo-6 - hydroxy - 4,5 - dihydro - 1,2,4 - triazin - 3 - ylthio)methyl - 3 - cephem - 4 - carboxylic acid,

7 - [2 - (2 - aminothiazol - 4 - yl) - 2 - methoxyiminoacetamido] - 3 - (4 - methyl - 5 - oxo - 6-hydroxy - 4,5 - dihydro - 1,2,4 - triazin - 3 - ylthio)methyl - 3 - cephem - 4 - carboxylic acid,

7 - [2 - (2 - aminothiazol - 4 - yl) - 2 - isobutoxyiminoacetamido] - 3 - (4 - methyl - 5 - oxo - 6-hydroxy - 4,5 - dihydro - 1,2,4 - triazin - 3 - ylthio)methyl - 3 - cephem - 4 - carboxylic acid,

7 - [2 - (2 - aminothiazol - 4 - yl) - 2 - hydroxyiminoacetamido] - 3 - (4 - *n* - butyl - 5 - oxo - 6-hydroxy - 4,5 - dihydro - 1,2,4 - triazin - 3 - ylthio)methyl - 3 - cephem - 4 - carboxylic acid and the corresponding lithium, potassium and sodium salts of each of the foregoing cephalosporin acids.

A preferred group of the present cephalosporin compounds are those represented by formula I wherein $R_4$ is methyl or ethyl; most preferred of that group are those compounds represented when $R_4$ is methyl.

Also preferred compounds of formula I are those represented when $R_1$ is hydrogen.

A further preferred group of the present compounds are those represented when $R_3$ is a $C_1$—$C_4$ alkyl group; the more preferred compounds within this group are those compounds represented when $R_3$ is methyl.

Another preferred group of the present cephalosporin compounds are those wherein R is hydrogen or an alkali metal cation; most preferred compounds within this group of the present cephalosporins are those compounds wherein, additionally, $R_2$ is hydrogen. These most preferred amino-acid cephalosporins of the present invention are those which particularly exhibit antibiotic activity. Compounds wherein R and $R_2$ are other than hydrogen are primarily useful as intermediates to the most active of the present compounds.

The novel cephalosporin carboxylic acids and their alkali metal salts are useful in combating infections in warm-blooded mammals when administered parenterally in non-toxic doses between about 10 and 500 mg./kg. of body weight. The actual dose employed will be varied in accordance with techniques well known by the medical community in the administration of cephalosporin antibiotics and will be determined by such factors as the nature and severity of the infection being treated, the frequency and duration of administration, the general condition of the patient and like factors.

The compounds can be used in a wide variety of oral or parenteral dosage forms solely or in admixture with other substances. The pharmaceutical compositions may be a mixture of 0.01 to 99% of a compound of formula I with a pharmaceutical carrier which can be a solid material or liquid material in which the compounds are dissolved, dispersed, or suspended. They can be in a unit dosage form. The solid compositions can take the form of tablets, powder, dry syrups, troches, granules, capsules, pills, suppositories, or like solid preparations. The liquid compositions can take the form of injections, ointments, dispersions, inhalant, suspensions, solutions, emulsions, syrups, or elixirs. They may be flavored and colored, and tablets, granules, and capsules may be coated.

All of the usual diluents (e.g. starch, sucrose, lactose, calcium carbonate, kaolin); bulking agents (e.g. lactose, sugar, salt, glycine, starch, calcium carbonate, calcium phosphate, kaolin, bentonite, talc, sorbitol); binders (e.g. starch, acacia, gelatin, glucose, sodium alginate, tragacanth, carboxymethylcellulose, syrup, sorbitol, polyvinylpyrrolidone); disintegrators (e.g. starch, agar, carbonates, sodium laurylsulfate); lubricant (e.g. stearic acid, talc, paraffin, boric acid, silica, sodium benzoate, polyethylene glycol, cacao oil, magnesium stearate, emulsifying agents (e.g. lecithin, sorbitan monooleate, acacia); suspending agents (e.g. sorbitol, methyl cellulose, glucose, or sugar syrup, gelatin, hydroxyethylcellulose, carboxymethylcellulose, aluminum stearate gel, hydrogenated fats); solvents (e.g. water, buffer, peanut oil, sesame oil, methyl oleate); preservatives (e.g. methyl or ethyl p-hydroxybenzoate, sorbic acid); edible coloring agents, aromatic substances, solubilizing agents, buffers, stabilizing agents, analgesics, dispersing agents, wetting agents, antioxidants, and the like can be used if the agents do not exert adverse effect on the compounds, according to the methods conventional in the art.

Compounds in salt form are soluble in water, and conveniently used as solution for intravenous, intramuscular, or subcutaneous injection. The compounds can be dissolved in aqueous or oily solvents for injection to give a solution in an ampoule, but generally, more prolonged storage is possible by making a vial preparation containing crystals, powder, microcrystals, or lyophilizate of the compound, and dissolving or suspending the drug before use with the said solvents for injection. The preparation may contain a preservative.

The compounds of the invention are useful in treating or preventing human or veterinary bacterial infections by administering to the human or animal subject an effective amount of the compound of formula I at a daily dose of e.g. 10 to 500 mg/kg body weight, e.g. an intervals of 3 to 12 hours. Preferably the compound is administered as a pharmaceutical composition as described above.

Compounds of formula I have been tested against typical Gram positive and Gram negative microorganisms to determine the range of their efficacy as antibiotics. The compounds have been found to be outstandingly potent. The following exemplary data is presented merely as an aid to the reader in understanding the broad range of effect of these compounds. The test method used was a typical standard media dilution test, and the data is presented as the minimum concentration of the compound, in mcg./ml., which was found to inhibit growth of the microorganism.

Compound A in the table below was 7-[2-(2-amino-4-thiazolyl)-2-methoxyiminoacetamido]-3-(4-methyl-5-oxo-6-hydroxy-4,5-dihydro-1,2,4-triazin-3-ylthio)methyl-3-cephem-4-carboxylic acid, sodium salt, and compound B was 7-[2-(2-amino-4-thiazolyl)-2-methoxyiminoacetamido]-3-(4-ethyl-5-oxo-6-hydroxy-4,5-dihydro-1,2,4-triazin-3-ylthio)methyl-3-cephem-4-carboxylic acid. Both compounds were the *syn* isomers.

| | Enterobacter aerogenes | Salmonella sp. | Serratia sp. | Proteus morganii | Staph. aureus | Streptococcus sp. |
|---|---|---|---|---|---|---|
| A | .25 | .25 | .25 | .25 | 2 | .125 |
| B | 2 | 1 | 1 | 1 | 4 | .06 |

| | Haemophilus influenza | Shigella sonnei | Escherichia coli | Klebsiella sp. |
|---|---|---|---|---|
| A | .125 | .25 | .25 | .125 |
| B | .06 | .5 | .5 | .06 |

The following preparations and examples are provided further to assist the reader to understand the compounds of formula I and the process by which they are made. In the preparations and examples below, the aminothiazole group is to be understood to be the *syn* isomer unless otherwise stated.

Preparation 1

To 20 ml. of water were added 3.46 g. of 7-formamido-3-acetoxymethyl-3-cephem-4-carboxylic acid (12.0 mmole) and 2.0 g. of 3-mercapto-4-methyl-5-oxo-6-hydroxy-4,5-dihydro-1,2,4-triazine (12.55 mmole). The resulting mixture was stirred, and 1N sodium hydroxide was added gradually until the pH remained at a constant 7.0. The resulting mixture was then stirred at about 55°C. for 26 hours. The resulting solution was concentrated to 20 ml. and acidified to pH 1.2 by addition with cooling of 3N hydrochloric acid. The resulting precipitate was filtered and immediately placed into a bell jar to dry under vacuum. The dried material was ground in a mortar and pestle (2.75 g.), and was triturated three times, each with 150 ml. of boiling isopropyl alcohol. The isopropyl alcohol solution was evaporated to dryness and the residue was triturated twice with 30 ml. of ethyl acetate. the insoluble material was filtered, washed with ethyl acetate, and dried to give 1.56 g. of 7-formamido-3-(4-methyl-5-oxo-6-hydroxy-4,5-dihydro-1,2,4-triazin-3-ylthio)methyl-3-cephem-4-carboxylic acid.

Preparation 2

The product from Preparation 1 (0.74 g.) was stirred in 12 ml. of dry methanol, and 1.5 ml. of concentrated hydrochloric acid were added during which time complete solution occurred. After a short period of time, a white solid began to precipitate. Stirring was continued for 1.7 hours, and the mixture became thick with a white precipitate. The precpitate was filtered and dried. The product (0.346 g.) was shown by TLC to be a highly pure sample of the hydrochloride of 7-amino-3-(4-methyl-5-oxo-6-hydroxy-4,5-dihydro-1,2,4-triazin-3-ylthio)methyl-3-cephem-4-carboxylic acid.

Preparation 3

To a suspension of 3.71 g. (10 mmole) of 7-amino-3-(4-methyl-5-oxo-6-hydroxy-4,5-dihydro-1,2,4-triazin-3-ylthio)methyl-3-cephem-4-carboxylic acid in 50 ml. of methylene chloride and 50 ml. of methanol was added 1.94 g. (10 mmole) of diphenyldiazomethane. The reaction mixture was allowed to stir overnight at room temperature. An additional 500 mg. of diphenyldiazomethane was then added. After two hours the reaction mixture was filtered and the filtrate was concentrated *in vacuo* to an oil. This product was dissolved in about 25 ml. of methylene chloride, and the resulting solution was added dropwise with stirring to hexane. A light yellow-brown amorphous solid formed. Filtration provided after drying 2.8 g. (52%) of benzhydryl 7-amino-3-(4-methyl-5-oxo-6-hydroxy-4,5-dihydro-1,2,4-triazin-3-ylthio)methyl-3-cephem-4-carboxylate: nmr ($CDCl_3$) $\delta$ 3.33 (s, 3, N—$CH_3$), 3.5 (m, 4, $C_2$—H, $C_3$—H), 4.86 (q, 2, $C_6$—H, $C_7$—H), 7.0 (s, 1, benzhydryl *CH*), 7.36 (s, 10, ArH).

Example 1

To a solution of 0.55 g. (1 mmole) of benzhydryl 7-amino-3-(4-methyl-5-oxo-6-hydroxy-4,5-dihydro-1,2,4-triazin-3-ylthio)methyl-3-cephem-4-carboxylate and 886 mg. (2 mmoles) of 2-(2-tritylamino-4-thiazolyl)-2-methoxyiminoacetic acid in 15 ml. of methylene chloride were added 208 mg. (1 mmole) of dicyclohexylcarbodiimide. After stirring the mixture six hours at room temperature, the reaction mixture was filtered. The filtrate was concentrated *in vacuo* to an oil which was then dissolved in ethyl acetate and washed successively with dilute sodium bicarbonate solution, water and brine. The ethyl acetate solution was then dried over anhydrous sodium sulfate and evaporated *in vacuo* to dryness to provide 800 mg. (83%) of benzhydryl 7-[2-(2-tritylamino-4-thiazolyl)-2-methoxyiminoacetamido] - 3 - (4 - methyl - 5 - oxo - 6 - hydroxy - 4,5 - dihydro - 1,2,4 - triazin - 3 - ylthio)methyl - 3-cephem - 4 - carboxylate as a red amorphous solid: nmr ($CDCl_3$, DMSO d-6) $\delta$ 3.3 (s, 3, N—$CH_3$), 3.7 (s, 2, $C_2$—H), 4.1 (s, 3, —$OCH_3$), 5.2 (d, 1, J=5.0 Hz, $C_6$—H), 5.8 (q, 1, J=5.0 and 8.0 Hz, $C_7$—H), 6.8 (s, 1, thiazolyl $C_5$—H), 7.0 (s, 1, benzhydryl CH) and 7.4 (s, 25, trityl and benzhydryl ArH).

Example 2

About 500 mg. of the product benzhydryl ester from Example 1 above was suspended in 10 ml. of 50% aqueous formic acid and heated on a steam bath for five minutes. The mixture was then stirred at 50 to 60°C. on a hot plate for 45 minutes. After allowing the mixture to cool to about 30°, it was filtered and the filtrate was concentrated *in vacuo* to an oily residue. Trituration of the residue with ethyl alcohol provided upon filtration a light brown amorphous solid which was then washed with methylene

chloride. Yield 161 mg. (56%). High pressure liquid chromatography shows the product to be very pure 7-[2-(2-amino-4-thiazolyl)-2-methoxyiminoacetamido]-3-(4-methyl-5-oxo-6-hydroxy-4,5-dihydro-1,2,4-triazin-3-ylthio)methyl-3-cephem-4-carboxylic acid: nmr (DMSO d-6) δ 3.29 (s, 3, N—$CH_3$), 3.65 (s, 2, $C_2$—H), 3.03 (s, 3, $OCH_3$), 4.10 (q, 2, $C_3$—H), 5.15 (d, 1, J=5.0 Hz. $C_6$—H), 5.77 (q, 1, J=5.0 and 8.0 Hz, $C_7$—H), 6.73 (s, 1, thiazolyl $C_5$—H), 7.20 (s, 2, $NH_2$), 9.58 (d, 1, J=8.0 Hz, side chain NH).

## Example 3

A mixture of 84 ml. of water, 42 ml. of acetone, 2.42 g. of sodium bicarbonate and 2.87 g. of 7-amino-3-(4-methyl-5-oxo-6-hydroxy-4,5-dihydro-1,2,4-triazin-3-ylthio)methyl-3-cephem-4-carboxylic acid hydrochloride was stirred and cooled in an ice-ethanol bath. When the solids had dissolved, a solution of 2.34 g. of 2-(2-chloroacetylamino-4-thiazolyl)-2-methoxyiminoacetic acid chloride in 42 ml. of acetone was added. The reaction mixture was stirred in the cooling bath for 95 minutes, and for 10 minutes more after the cooling bath was removed. The acetone was evaporated in a rotary evaporator at room temperature, 50 ml. of ethyl acetate was added to the residue and the mixture was acidifed to pH 2.2 with dilute hydrochloric acid. The solids were filtered off, washed thoroughly with water and dried under vacuum at room temperature to obtain 3.76 g. of the product of Example 2 in the N-chloroacetyl form.

NMR analysis in DMSO d-6 showed a one proton singlet at δ 7.44 ppm.; the Cl analysis was 5.52%; calculated Cl analysis, for $C_{20}H_{19}N_8O_8S_3Cl$, was 5.62%.

## Example 4

A 4.4 g. portion of 2-(2-tritylamino-4-thiazolyl)-2-methoxyiminoacetic acid was converted to the acid chloride, and dissolved in acetone. The acetone solution was added to an ice-cold suspension of 4-nitrobenzyl 7-amino-3-(4-methyl-5-oxo-6-hydroxy-4,5-dihydro-1,2,4-triazin-3-ylthio)methyl-3-cephem-4-carboxylate hydrochloride, 3.86 g., and 4.77 g. of sodium bisulfite in 200 ml. of acetone. After the addition, the ice bath was removed and the reaction mixture was stirred for 20 hours. The solids were then separated by filtration, dissolved in ethyl acetate, and washed successively with sodium bicarbonate solution, 1N hydrochloric acid, water and brine. The ethyl acetate solution was then dried over sodium sulfate and concentrated to a yellow oil. The oil was dissolved in dichloromethane and filtered, and the filtrate was purified by passage through a pad of silica gel, eluting first with dichloromethane and then with 1/9 ethyl acetate/dichloromethane. A total of 2.4 g. of 4-nitrobenzyl 7-[2-(2-tritylamino-4-thiazolyl)-2-methoxyiminoacetamido]-3-(4-methyl-5-oxo-6-hydroxy-4,5-dihydro-1,2,4-triazin-3-ylthio)methyl-3-cephem-4-carboxylate, mp 140—146°C., was obtained.

The 4-nitrobenzyl ester group of the above intermediate product was removed by hydrogenation, obtaining 85 mg. of the corresponding acid from 1.5 g of the above intermediate. The acid was suspended in 5 ml. of 50% formic acid. The suspension was heated briefly on a steam bath at 60—80°C., and the mixture was agitated gently for 10 minutes. The reaction mixture was then cooled and filtered. The addition of water to the filtrate gave a precipitate, which was removed by filtration. The filtrate was concentrated under vacuum to obtain a yellow oil, which was dissolved in a few ml. of denatured ethanol. Diethyl ether was added until precipitation was complete. The suspension was filtered to obtain 18 mg. of product identical to the product of Example 2.

## Example 5

A 30 mg. portion of the product of Example 3 was dissolved in 1 ml. of a solvent, a small portion of base was added, and then 7.3 mg. of thiourea was added. The reaction mixture was then stirred at room temperature, and the reactions were followed by thin layer chromatography. The product of Example 2 was identified in essentially pure state after reaction times from 5 to 24 hours in various instances. The solvents and bases used successfully in the above process were as follows:

(a) dimethylformamide plus triethylamine
(b) dimethylformamide plus sodium acetate
(c) water plus sodium bicarbonate
(d) pyridine (no additional base)

## Example 6

A 10 g. portion of 4-nitrobenzyl 7-[2-(2-tritylamino-4-thiazolyl)-2-methoxyiminoacetamido]-3-bromomethyl-3-cephem-4-carboxylate was stirred at ambient temperature in 40 ml. of dimethylformamide with 2.5 g. of 3-mercapto-4-methyl-5-oxo-6-hydroxy-4,5-dihydro-1,2,4-triazine for 6 hours. An additional 0.5 g. of the thiol was added and the mixture was stirred for 16 hours more. The reaction mixture was then poured into a 1:1 mixture of ethyl acetate and dilute aqueous hydrochloric acid, and the organic layer was washed successively with dilute hydrochloric acid, water, dilute aqueous sodium bicarbonate and brine. The organic layer was then filtered and concentrated under vacuum to a foam. The yield was 10.5 g of crude product, which was purified by chromatography over silica gel to obtain a 1.5 g. portion of 4-nitrobenzyl 7-[2-(2-tritylamino-4-thiazolyl)-2-methoxyiminoacetamido] - 3 - (4 - methyl - 5 - oxo - 6 - hydroxy - 4,5 - dihydro - 1,2,4 - triazin - 3 - ylthio)methyl - 3-cephem - 4 - carboxylate. A 100 mg. portion of the above product was added to 5 ml. of 50% aqueous formic acid and heated on the steam bath with agitation for 10 minutes. The mixture was then cooled and filtered, and the filtrate was concentrated under vacuum to an oil. The oil was triturated with denatured ethanol and the solid isolated by filtration to obtain 42 mg. of the product of Example 2 in the form of the 4-nitrobenzyl ester.

Hydrogenation of the above product produces the product of Example 2 in essentially pure form.

Example 7

An acylation substantially like the process of Example 3 was carried out, except that the reaction was at ambient temperature. The starting materials were 1.85 g. of 7-amino-3-(4-methyl-5-oxo-6-hydroxy-4,5-dihydro-1,2,4-triazin-3-ylthio)methyl-3-cephem-4-carboxylic acid and 1.58 g. of 2-(2-chloroacetylamino-4-thiazolyl)-2-methoxyiminoacetic acid chloride as the *anti* isomer. A 1.06 g. portion of the product obtained was reacted at room temperature with thiourea and sodium bicarbonate as described in Example 7 to obtain 0.55 g. of 7-[2-(2-amino-4-thiazolyl)-2-methoxyiminoacetamido] - 3 - (4 - methyl - 5 - oxo - 6 - hydroxy - 4,5 - dihydro - 1,2,4 - triazin - 3 - ylthio)methyl-3-cephem-4-carboxylic acid, which was primarily in the form of the *anti* isomer, readily identified by nmr which showed a one-proton singlet indicating the C—5 proton of the thiazole ring, at $\delta$ 7.98 ppm.

Example 8

A 1.9 g. portion (7 mmoles) of 7-aminocephalosporanic acid was suspended in 70 ml. of dry acetonitrile and 7 ml. of N,O-bis(trimethylsilyl)acetamide was added. The mixture was stirred vigorously and was then cooled in ice-acetone under a nitrogen atmosphere, and 7 mmoles of 2-(2-tritylamino-4-thiazolyl)-2-methoxyiminoacetic acid chloride was added. After the reaction was stirred for 10 minutes the ice bath was removed and stirring was continued for 1 hour. The solution was concentrated to half its volume under vacuum and poured into 200 ml. of water. After 15 minutes of stirring, the aqueous mixture was acidified to pH 1.7 with 20% hydrochloric acid, and the intermediate product was separated by filtration to obtain 4.5 g. of 7-[2-(2-tritylamino-4-thiazolyl)-2-methoxyiminoacetamido]-cephalosporanic acid.

The above product was deblocked with formic acid as described in Example 2, and a 113 mg. portion of it was combined with 70 mg. of 3-mercapto-4-methyl-5-oxo-6-hydroxy-4,5-dihydro-1,2,4-triazine and 38 mg. of sodium bicarbonate in 6 ml. of pH 6.4 buffer. The aqueous mixture was maintained at pH 6.2 and was held at 55°C. for 22 hours. The reaction was monitored by thin layer chromatography and the product was identified by TLC as identical with the product of Example 2.

## Claims

1. A cephalosporin compound of the formula

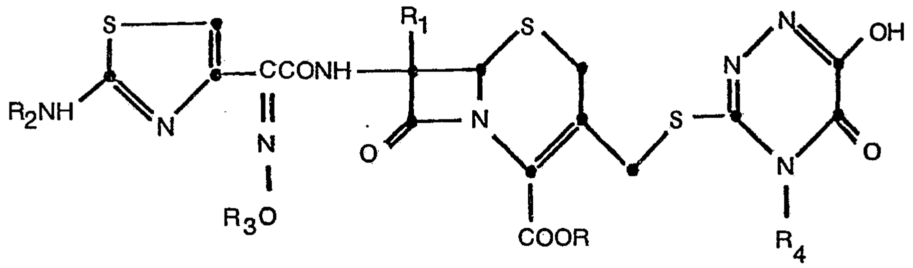

I

wherein R is hydrogen, an alkali metal cation, or a carboxylic acid protecting group; $R_1$ is hydrogen or methoxy; $R_2$ is hydrogen or an amino protecting group; $R_3$ is hydrogen or $C_1$—$C_4$ alkyl; and $R_4$ is $C_1$—$C_4$ alkyl.

2. A compound of claim 1 wherein $R_4$ is methyl.

3. A compound of any of claims 1—2 wherein $R_1$ is hydrogen.

4. A compound of claim 3 wherein $R_3$ is methyl.

5. A compound of either of claim 3 or 4 wherein R is hydrogen or an alkali metal cation.

6. A compound of claim 5 wherein $R_2$ is hydrogen.

7. The compound of claim 1 which is 7-[2-(2-amino-4-thiazolyl)-2-methoxyiminoacetamido]-3-(4-methyl-5-oxo-6-hydroxy-4,5-dihydro-1,2,4-triazin-3-ylthio)methyl-3-cephem-4-carboxylic acid.

8. The compound of claim 1 which is 7-[2-(2-amino-4-thiazolyl)-2-methoxyiminoacetamido]-3-(4-methyl-5-oxo-6-hydroxy-4,5-dihydro-1,2,4-triazin-3-ylthio)methyl-3-cephem-4-carboxylic acid, sodium salt.

9. A compound of formula I as defined in any of claims 1—8 which is the syn isomer.

10. A pharmaceutical formulation comprising a pharmaceutically-acceptable inert carrier and a compound of formula I as defined in any of claims 1—9.

11. A compound of formula I as defined in any of claims 1—9 for use in the treatment or prevention of human or veterinary bacterial infections.

12. A process for preparing a compound of formula I as defined in claim 1 which comprises reacting a compound of the formula

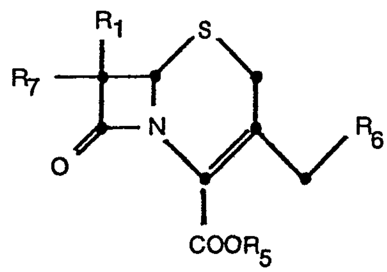

II

wherein $R_5$ is hydrogen, an alkali metal cation, or a carboxy-protecting group; $R_6$ is acetoxy, chloro, bromo, iodo or a group of the formula

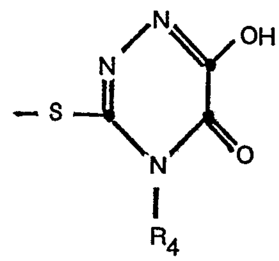

III

and $R_7$ is amino, amino substituted with a silyl amino-protecting group, amino hydrohalide or a group of the formula

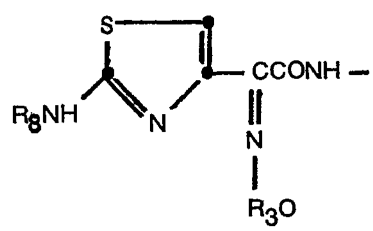

IV

wherein $R_8$ is hydrogen or an amino-protecting group; in either order with a thiol of the formula

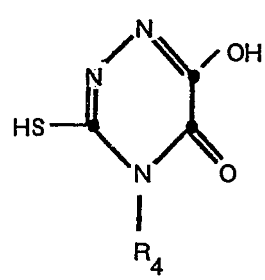

V

when $R_6$ is other than a group of formula III, and with an acylating agent of the formula

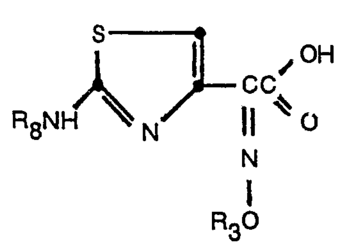

VI

which is in the form of the chloride, bromide or an activating ester when $R_7$ is other than a group of formula IV; optionally cleaving the amino-protecting and carboxy-protecting groups; and, when R does not represent a carboxy-protecting group, recovering the compound of formula I as the acid or the alkali metal salt.

**Revendications**

1. Composé de céphalosporine de formule

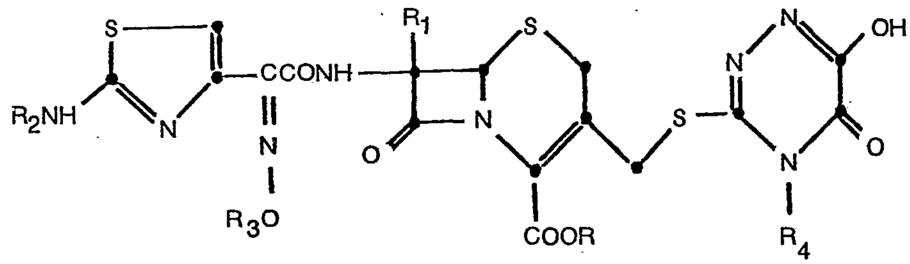

I

où R est un hydrogène, un cation de métal alcalin, ou un groupe protecteur d'acide carboxylique; $R_1$ est un hydrogène ou un méthoxy; $R_2$ est un hydrogène ou un groupe protecteur d'amino; $R_3$ est un hydrogène ou un alcoyle en $C_1$ à $C_4$; et $R_4$ est un alcoyle en $C_1$ à $C_4$.

2. Composé de la revendication 1 où $R_4$ est un méthyle.

3. Composés de l'une des revendications 1 ou 2 où $R_1$ est un hydrogène.

4. Composé de la revendication 3 où $R_3$ est un méthyle.

5. Composé de l'une des revendications 3 ou 4 où R est un hydrogène ou un cation de métal alcalin.

6. Composé de la revendication 5 où $R_2$ est un hydrogène.

7. Composé de la revendication 1 qui est l'acide 7-[2-(2-amino-4-thiazolyl)-2-méthoxyiminoacétamido] - 3 - (4 - méthyl - 5 - oxo - 6 - hydroxy - 4,5 - dihydro - 1,2,4 - triazin - 3 - ylthio)méthyl-3-cephem-4-carboxylique.

8. Composé de la revendication 1 qui est le sel de sodium de l'acide 7-[2-(2-amino-4-thiazolyl)-2 - méthoxyiminoacétamido] - 3 - (4 - méthyl - 5 - oxo - 6 - hydroxy - 4,5 - dihydro - 1,2,4 - triazin-3-ylthio)méthyl-3-cephem-4-carboxylique.

9. Composé de formule I tel que défini dans l'une quelconque des revendications 1 à 8 qui est l'isomère syn.

10. Formulation pharmaceutique comprenant un support inerte pharmaceutiqement acceptable et un composé de formule I tel que défini dans l'une quelconque des revendications 1—9.

11. Composé de formule I tel que défini dans l'une quelconque des revendications 1—9 aux fins d'application dans le traitement ou la prévention des infections bactériennes chez l'homme ou l'animal.

12. Procédé de préparation d'un composé de formule I tel que défini dans la revendication 1 qui implique de faire réagir un composé de formule

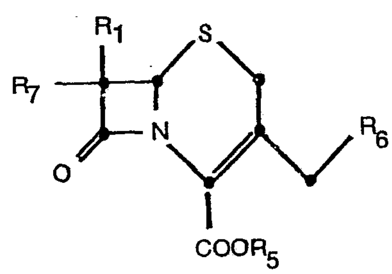

II

où $R_5$ est un hydrogène, un cation de métal alcalin, ou un groupe protecteur de carboxy; $R_6$ est un acétoxy, un chloro, un bromo, un iodo ou un groupe de formule

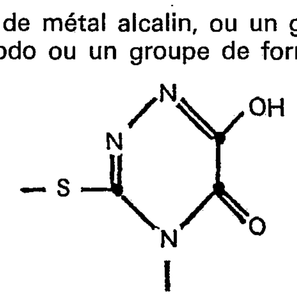

III

et $R_7$ est un amino, un amino substitué par un groupe silyle protecteur d'amino, un halohydrate d'amino ou un groupe de formule

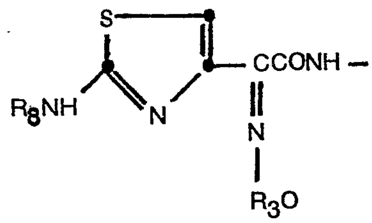

IV

où $R_8$ est un hydrogène ou un groupe protecteur d'amino; dans un ordre quelconque avec un thiol de formule

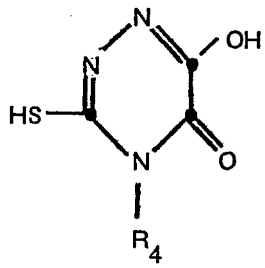

V

où $R_8$ est différent d'un groupe de formule III, et avec un agent acylant de formule

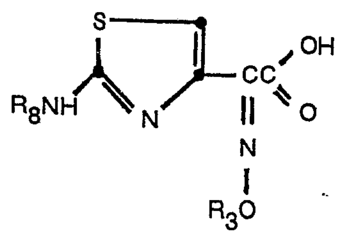

VI

qui est sous la forme de chlorure, du bromure ou d'un ester activant lorsque $R_7$ est différent d'un groupe de formule IV; éventuellement en clivant les groupes protecteurs d'amino et protecteurs de carboxy; et, lorsque R ne représente pas un groupe protecteur de carboxy, en recueillant le composé de formule I sous la forme de l'acide ou du sel de métal alcalin.

**Patentansprüche**

1. Cephalosporinverbindung der Formel

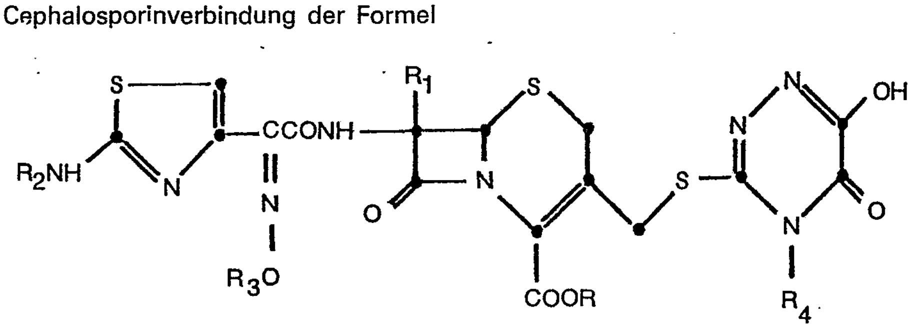

I,

worin R Wasserstoff, ein Alkalimetallkation oder eine Carbonsäureschutzgruppe bedeutet, $R_1$ Wasserstoff oder eine Methoxygruppe ist, $R_2$ Wasserstoff oder eine Aminoschutzgruppe ist, $R_3$ Wasserstoff oder eine Alkylgruppe mit 1 bis 4 Kohlenstoffatomen darstellt und $R_4$ für eine Alkylgruppe mit 1 bis 4 Kohlenstoffatomen steht.

2. Verbindung nach Anspruch 1, dadurch gekennzeichnet, daß $R_4$ Methyl bedeutet.

3. Verbindung nach einem der Ansprüche 1 bis 2, dadurch gekennzeichnet, daß $R_1$ Wasserstoff ist.

4. Verbindung nach Anspruch 3, dadurch gekennzeichnet, daß $R_3$ für Methyl steht.

5. Verbindung nach einem der Ansprüche 3 oder 4, dadurch gekennzeichnet, daß $R_4$ Wasserstoff oder ein Alkalimetallkation ist.

6. Verbindung nach Anspruch 5, dadurch gekennzeichnet, daß $R_2$ Wasserstoff darstellt.

7. 7-[2-(2-Amino-4-thiazolyl)-2-methoxyiminoacetamido]-3-(4-methyl-5-oxo-6-hydroxy-4,5-dihydro-1,2,4-triazin-3-ylthio)methyl-3-cephem-4-carbonsäure nach Anspruch 1.

8. 7-[2-(2-Amino-4-thiazolyl)-2-methoxyiminoacetamido]-3-(4-methyl-5-oxo-6-hydroxy-4,5-dihydro-1,2,4-triazin-3-ylthio)methyl-3-cephem-4-carbonsäurenatriumsalz nach Anspruch 1.

9. Verbindung der Formel I gemäß einem der Ansprüche 1 bis 8, dadurch gekennzeichnet, daß es sich hierbei um das syn.-Isomer handelt.

10. Pharmazeutische Formulierung aus einem pharmazeutisch unbedenklichen inerten Träger und einem Wirkstoff, dadurch gekennzeichnet, daß sie als Wirkstoff eine Verbindung der Formel I gemäß einem der Ansprüche 1 bis 9 enthält.

11. Verwendung einer Verbindung der Formel I nach einem der Ansprüche 1 bis 9 zur Behandlung oder Verhinderung von Bakterieninfektionen bei Mensch oder Tier.

12. Verfahren zur Herstellung einer Verbindung der Formel I gemäß Anspruch 1, dadurch gekennzeichnet, daß man eine Verbindung der Formel

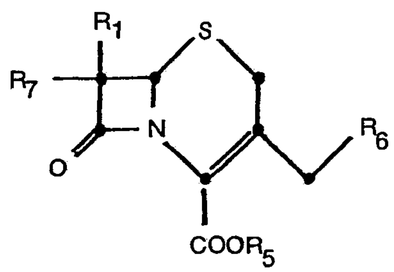

II,

worin $R_5$ Wasserstoff, ein Alkalimetallkation oder eine Carbonsäureschutzgruppe bedeutet, $R_6$ eine Acetoxygruppe, Chlor, Brom, Iod oder eine Gruppe der Formel

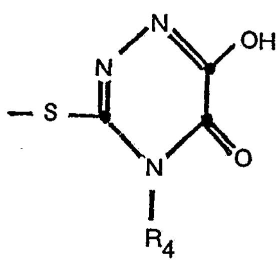

III

darstellt und $R_7$ eine Aminogruppe, eine durch eine Silylaminoschutzgruppe substituierte Aminogruppe, Aminohydrohalogenid oder eine Gruppe der Formel

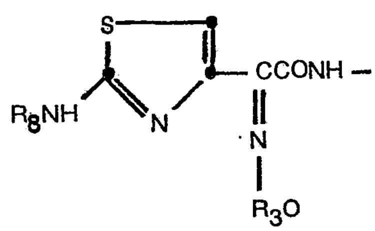

IV,

ist, worin $R_8$ für Wasserstoff oder eine Aminoschutzgruppe steht, in beliebiger Reihenfolge mit einem Thiol der Formel

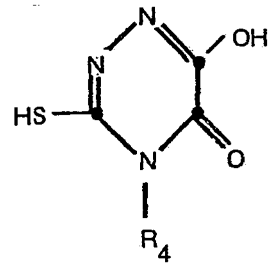

V,

wenn $R_6$ eine andere Bedeutung als die einer Gruppe der Formel III hat, und mit einem Acylierungsmittel der Formel

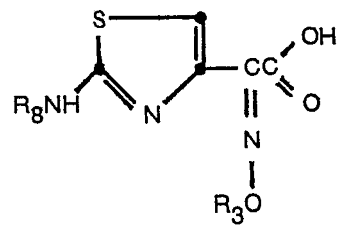

VI,

das in Form eines Chlorids, Bromis oder eines aktivierten Esters vorliegt, wenn $R_7$ eine andere Bedeutung als die einer Gruppe der Formel IV hat, umsetzt, gegebenenfalls die Aminoschutzgruppen und Carboxyschutzgruppen abspaltet, und, falls R keine Carbonsäureschutzgruppe bedeutet, die Verbindung der Formel I als Säure oder Alkalisalz gewinnt.